Europäisches Patentamt

European Patent Office

Office européen des brevets

(11) Publication number: **0 265 951 B1**

# EUROPEAN PATENT SPECIFICATION

(45) Date of publication of patent specification: **26.08.92**　(51) Int. Cl.⁵: **A61K 9/20**, A61K 9/16, C01B 25/32

(21) Application number: **87115914.1**

(22) Date of filing: **29.10.87**

(54) Granular tricalcium phosphate compositions suitable for direct compression tableting.

(30) Priority: **31.10.86 US 925761**

(43) Date of publication of application:
**04.05.88 Bulletin  88/18**

(45) Publication of the grant of the patent:
**26.08.92 Bulletin  92/35**

(84) Designated Contracting States:
**DE FR GB NL**

(56) References cited:

**CHEMICAL ABSTRACTS, vol. 88, no. 22, 29th May 1978, page 377, abstract no. 158374b, Columbus, Ohio, US; R.C. SHAH et al.: "Colored granules for compression coating", & DRUG. DEV. IND. PHARM. 1977, 3(4), 347-9**

(73) Proprietor: **RHONE-POULENC BASIC CHEMICALS CO.
1 Corporate Drive
Shelton, CT 06484(US)**

(72) Inventor: **Chu, Chihang Raymond
650 Warburton Avenue
Yonkers New York 10701(US)**

(74) Representative: **Jaeger, Klaus, Dipl.-Chem. Dr. et al
Jaeger, Steffens & Köster Patentanwälte Pippinplatz 4a
W-8035 München-Gauting(DE)**

**Description**

The present invention relates to a process for preparing a wet granulatable compressible tricalcium phosphate and to wet granulatable tricalcium phosphate compositions which are suitable for direct compression tableting and to tablets produced by direct compression.

BACKGROUND OF THE INVENTION

In compressing a dry particulate material into tablets, the direct compression technique is the most desirable. It employs the fewest steps and, in the case of pharmaceutical tablets containing sensitive or unstable active materials, minimizes the contact of the active ingredient with water, organic solvents or other conditions tending to adversely affect the stability thereof.

Tableting material for dry direct compression must be compressible into a tablet form, and must produce strong tablets with good tablet surfaces and good strength, particularly under the stress of automatic tableting equipment. The direct compression vehicle must be flowable into the dies of high speed tableting machines without bridging as so often occurs with fine powders. Since the amount of active material contained in a tablet is based on the weight of the tablet, weight variations caused by improper flow cannot be tolerated. The vehicle must also have good stability under normal ambient conditions so that it can be effectively compressed.

Most powdered dry materials are impractical for direct compression tableting, particularly in automatic tableting equipment because of compressive strength and/or flow problems. Some of these powders can be granulated using a wet granulation process, with or without the addition of an adhesive substance. The moistened powder is converted into a crumbly mass which is forced through a screen to reduce the material to a grain-like structure of small granules. It is then dried, milled and sieved.

The powder can also be dry granulated by precompressing the dry powder, such as, into slugs or passing the material between two compressing rollers followed by breaking the material into granular particles of uniform size.

Tricalcium phosphate powder (98% through 325 mesh) is not adaptable for direct compression tableting. A successful method for preparing tricalcium phosphate for direct compression tableting is through roller compaction. This places a limit on the applicability of the product as far as formulation is concerned since the formulator cannot adjust the ingredients in the excipient (color, lubricants, disintegrants) prior to roller compaction as the roller compaction is generally conducted by the supplier of the excipient.

Pharmaceutical manufacturers would like to wet granulate tricalcium phosphate since they can add their own ingredients during the wet granulation process and then tablet the material. In addition, wet granulated excipients are more desirable since tablet size in general is smaller, the tablets are harder and have a better appearance. Tricalcium phosphate powder cannot be easily wet granulated as it does not wet easily, uses more binder and does not dry as completely under moderate conditions. Ultrafine tricalcium phosphate (about 5 $\mu$m particle size) is used as a flow control agent in wet granulated materials. Presently available roller compacted tricalcium phosphate excipients are not adaptable for wet granulation as the particle size of the roller compacted tricalcium phosphate is suffcently large that further increase in particle size through wet granulation would make the product unsuitable for direct compression tableting for pharmaceuticals.

SUMMARY OF THE INVENTION

In accordance with the present invention there is provided a process for preparing a wet granulatable compressible tricalcium phosphate said process comprises compacting tricalcium phosphate and classifying the compacted tricalcium phosphate to such a particle size that the classified particles have a size within the range of less than about 10% larger than about 420 $\mu$m, from 40% to 70% larger than 149 $\mu$m and smaller than 420 $\mu$m and less than about 25% smaller than about 25 $\mu$m.

In accordance with the present invention there is furthermore provided a wet granulatable tricalcium phosphoate composition having a particle size range of less than about 10% larger than about 420 $\mu$m, from 40% to 70% larger than 149 $\mu$m and smaller than 420 $\mu$m and less than about 25% smaller than about 25 $\mu$m.

The compacted or densified tricalcium phosphate as obtained by the process of the invention and the composition of the invention can be wet granulated easily with fast wetting and improved drying. The wet granulated tricalcium phosphate exhibits good compressability, flowability and whiteness.

DETAILED DESCRIPTION OF THE INVENTION

The wet granulatable tricalcium phosphate of the invention can be formed by compacting and comminuting tricalcium phosphate.

The tricalcium phosphate is compacted by the static application of an amount of force sufficient to densify the tricalcium phosphate such as by using an hydraulic press. Preferably, the densified tricalcium phosphate is compacted by feeding a powder mass of tricalcium phosphate under controlled volume flow into the nip of the counterrotating rolls of a roller compactor. The roller compactor can be of the type sold under the trademark CHILSONATOR as described in U.S. Patent No. 3,255,285.

In an hydraulic press, the force applied can range from about 325 kilograms (716 pounds) to about 700 kilograms (1543 pounds). In a roller compactor, the force employed for the compaction step can vary from about 714 kilograms force/linear centimeter roll width (4,000 pounds/linear inch) to about 3214 kilograms force/linear centimeter roll width (18,000 pounds/linear inch) or higher. The preferred range in applied force for compaction in a roller compactor is from about 1428 kilograms force/linear centimeter roll width (8,000 pounds/linear inch) to about 2,500 kilograms force/linear centimeter roll width (14,000 pounds/linear inch). The compaction step can be carried out at a high or low rate of application of pressure.

The thickness of the compacted material in the case of the hydraulic press is determined by the amount of powder charged. In the case of the roller compactor, the thickness of the ribbon produced is determined by the rate of feed of powder into the nip between the rolls, as well as the configuration of the rolls and the force applied to the rolls.

The particle size of the fine powder used as the feed material varies from 0.5 $\mu$m to 75 $\mu$m with a majority of the particles being within the range of from 25 to 75 $\mu$m. The bulk density of the fine particle feed to the compacting process of the present invention ranges from 0.24 to 0.40 g/cc (from about 15 to about 25 lbs/ft$^3$).

The surface of the rolls or plates applying the mechanical force can be scored to give corrugated, patterned or briquetted compacted product of any desired shape. Corrugations facilitate the flow of the fine feed into the nip of the rolls.

Compaction can be carried out at low relative humidity and ambient temperature. The compaction step in exerting mechanical pressure on the fine particles of the tricalcium phosphate by means of rolls increases the temperature about 10°C to about 30°C. Chilling the pressure rolls is unnecessary unless discoloration of the compacted material commences.

The compacted product is then comminuted by any means appropriate to produce the particle sizes which can effectively be granulated in accordance with the invention, such as by grinding.

Grinding mills such as those manufactured by the Fitzpatrick Company, Elmhurst, Illinois; Pulverizing Machinery Company, Summit, New Jersey; and Raymond Division of Combustion Engineering Company, Stamford, Connecticut, can be used to prepare the desired sized granules from the compacted sheet, ribbon, flakes or chips.

Optionally, the compacted ribbon or sheet of the blend can be pre-broken up into flakes, chips, slices or pieces by standard cutting machines prior to grinding. It is convenient to attach a rotating set of cutting knives just below the compacting chamber of a continuous roller compactor so that the ribbon of compacted material is immediately broken up into pieces varying in size from about 5 millimeters to 50 millimeters. The speed of rotation of the prebreaking knives varies from about 40 to about 1000 rpm, thus determining the size of the pieces.

After comminuting, the product can be classified to provide the desirable particle size for wet granulation. Any appropriate screening or sieving device including screens, perforated plates, cloth and the like, and air separators and the like can be used if appropriate classification of particles can be obtained. The large particles can be recycled to the compactor for further processing. For effective wet granulation, the particles must be sufficiently small such that after wet granulation the particles can be used in the final use area. In the case of wet granulated material for use in direct compression tableting, the densified tricalcium phosphate has a particle size distribution of less than about 25% and preferably less than about 20% through 325 mesh, i.e., smaller than 44 $\mu$m; from 40% to 70% and preferably from 45% to 65% on 100 mesh i.e. larger than about 149 $\mu$m; and a maximum of 10% and preferably a maximum of 5% on 40 mesh, i.e., larger than about 420 $\mu$m. The particle size distribution is directly related to the wet granulatable property of the tricalcium phosphate. The presence of a limited quantity of fines is necessary for good wet granulatability. The loose bulk density of the densified tricalcium phosphate for preparing pharmaceutical tableting material preferably ranges from 0.70 to 0.90 and preferably from 0.75 to 0.85 g/cc (44 to 56 and preferably 47 to 53 lbs/ft$^3$); and tapped (50 times) from 0.9 to 1.2 g/cc and preferably from 1.0 to 1.1 g/cc (56 to 75 lbs/ft$^3$ and preferably from 62 to 69 lbs/ft$^3$).

The compacted and comminuted tricalcium phosphate can then be granulated into granules having a particle size at least 90% above 44 μm or granules which can be comminuted into the desired particle size range for tableting. Binders and processes which are presently used to agglomerate can be used. The granulation can be conducted with any known binder such as starches, modified or unmodified; gums such as acacia, locust bean, guar and the like; synthetic polymers such as polyvinylpyrrolidone; cellulose derivatives such as cellulose ethers including, for example, hydroxypropyl methylcellulose, hydroxypropyl cellulose, gelatins including gelatin hydrolysates; and the like. The end use of the excipient can dictate type, quality and purity of the binders.

The starch can be any starch or blends thereof, modified or unmodified, which is soluble (swellable) under the conditions of use. The starch can be derived from cereal grains, i.e., corn, sorghum, rice and preferably wheat; tubers or roots such as cassava (tapioca), potato or arrowroot and the pith from sago palm. The starch can be unmodified and not treated to reduce viscosity (thin boiling) or pregelatinized to improve the cold water solubility.

The amounts of binder used can vary depending on the binding capacity and viscosity of the binding solution. For example, starch solutions may require less binder than less viscous acacia gum solutions. The ability of a binder to flow within the porous surface of the tricalcium phosphate can be a function of viscosity. Optimum amounts of binder can be determined without undue experimentation by one of ordinary skill in the art. Amounts of binder can range from 2% to 10% based on the weight of the tricalcium phosphate.

Prior to wet granulation, the tricalcium phosphate can be admixed with other ingredients including binders, disintegrants, colorants, flavors, diluents, and active materials as desired.

Granulation processes using binders include such known techniques as fluid bed granulation (preferred), granulation (followed by extrusion) and high speed high shear mixing. Granulating procedures which can severely abrade the product are to be avoided as the particle structure can be seriously affected. The product can be dried using known techniques.

The wet granulated product can be then classified by any means appropriate to produce the particle sizes which can effectively be used in direct compression tableting. Any appropriate screening or sieving device including screens, perforated plates, cloth and the like, and air separators and the like can be used if appropriate classification of particles can be obtained. Mild milling can be used to reduce the size of large granules, preferably isolated prior to milling.

If milling is required, it is preferred that particles larger than 840 μm (20 mesh) are separated first and then milled.

Compositions which are too fine or which contain a significant amount of fines lack the flowability into the tableting dies needed to form tablets of uniform weight and thickness in a tableting machine.

Fines of smaller than 44 μm can be included in the product if not excessive, i.e. does not prevent proper flow into the direct compression dies (less than about 10%), or separated by particle classification means such as those discussed above.

The wet granulated tricalcium phosphate particles of the invention preferably have granules of at least about 90% and preferably at least 95% above 44 μm (325 mesh), and preferably at least 80% above 74 μm (200 mesh). The upper limit on particle size is that size which can be processed in a tableting machine. The particle size is less than about 10% greater than and preferably less than about 5% greater than 420 μm (40 mesh) for flowability into the tableting machine. The lower limit on particle sizes is greater than that which is flowable into the tableting machine.

Following classification, the particles having the desired particle size range are segregated for further processing. The fine and large particles can be recycled for further processing.

The products of the invention can be made into tablets by direct compression using well-known techniques. Prior to tableting, the active ingredient(s) along with other ingredients such as binders, disintegrants, colorants, flavors, diluents and any other materials normally used in tablets can be blended with the granules. A lubricant is usually blended last to coat the surface of the particles and provide exterior lubrication.

After mixing, the blend can be formed into a tablet by dry, direct compression. Either single hydraulic units or multiple anvil high-speed rotary tableting units can be used as known in the industry. The tablets can be formed in any desired shape such as round tablets or dry capsules with equivalent results. The composition of the present invention can be compressed at a high rate of compression in a rotary tableting machine as well as a low rate of compression utilizing a single tablet hydraulic unit with effective results.

Tablets can be prepared using the products of the invention solely as the excipient or in blends with other excipients such as microcrystalline cellulose (usually up to about 20%), roller compacted tricalcium phosphate, lactose and the like. For example, the product of the invention can be blended with from zero to 95% roller compacted tricalcium phosphate. Preferably, the amount of the product of the invention used exceeds 40% and more preferably about 50%. Blends may also be desirable in providing a shorter tablet disintegration time. Depending on the binder used, tablet disintegration times (as tested in water) showed increases which may be disadvantageous for a product directed to a particular end use. In theory, the binder may be coating the tablet with a layer which prevents water from entering the tablet, wetting the tablet disintegrant (if used) thereby preventing it from swelling to break the tablet. The use of more water or acid soluble binders or of blends of the product of the invention with other excipients may be useful in adjusting the rate of tablet disintegration.

The product of the invention can also be used as a filler in capsules or as an absorbent for oily material such as fragrances.

The invention is illustrated in the Examples which follow. All percentages and ratios are by weight unless otherwise stated. As used herein all sieve sizes are U.S. Standard Sieve Series.

EXAMPLE 1

Part 1. Densification of tricalcium phosphate (TCP) powder

USP/FCC grade of tricalcium phosphate with a bulk density of about 0.3 g/ml, manufactured by Stauffer Chemical Company, was compacted in a Fitzpatrick Chilsonator™ system provided with rolls of 3.75 centimeters (1.5 inches) in width and 20 centimeters (8 inches) in diameter. The rolls had a sine-wave surface and were separated with a roll gap of 0.05 centimeter (0.020 inches). The powder mixture was fed to the Chilsonator compactor by a screw feeder attached to the compactor unit and compacted by the rolls. One roll was hydraulically forced against the other with a pressure of 68.947 bar gauge (1000 psig). The effective force applied was 1485 kilograms/linear centimeter roll width (8300 pounds/linear inch). The rolls had a rotational speed of 7 rpm. The feed material was a fine and fluffy powder. The compacted product, which appeared as short "sticks", was hand fed to a Fitzpatrick Fitzmill Homoloid™ machine, Model JT. The mill operated at 750 rpm and was fitted with a 10 mesh (U.S. Standard Sieve Series) discharge screen. The milled product, with a bulk density of about 1.0 g/ml, appeared as a densified powder containing some granular particles.

Part 2: Fluid-bed granulation of densified TCP powder.

An Aeromatic™ batch fluid bed granulator, Model STERA-1, was used to granulate the roller compactor-densified TCP powder. 700 grams of the TCP was fluidized in the granulator. A starch paste was then sprayed via an atomizing nozzle into the fluidized bed. The starch paste was prepared by combining 13 grams of corn starch powder (National Starch's Purity 21 grade) with 156 grams of 25°C water and 40 grams of 80°C water. The mixture was then heated to 100°C to thicken to a paste consistency. Granulation occurred rapidly as a total of 164 grams of the starch paste was added. After the starch addition, the granular product continued to be fluidized and dried for 1 hour with 60°C air. This product had a calculated starch content of 1.4% by weight.

EXAMPLES 2-9

Densification of tricalcium phosphate was conducted on a plant scale using the procedure of Example 1 with a roller compactor having a roller width of 30.5 centimeters (12 inches) and a roller diameter of 30.5 centimeters (12 inches). Listed hereinafter are the conditions of compacting and classification. (Roll pressure and horizontal screw speed are averages.) Also listed are the results achieved;

| Sample | 1 | 2 |
|---|---|---|
| Roll Pressure, bar | 113,8 - 117,3 | 100,0* |
| Horizontal Screw, rpm | 21 | 28* |
| Vertical Screw, rpm | 800 | 800 |
| Mill Speed, rpm | 2250 | 2250 |
| Mill Screen, mm | 2,01 | 2,01 |
| Scalping Screen, Mesh | 40 | 40 |
| Product Screen | Blind | Blind |

*Average of 4 runs.

| Sievings | | | | | | |
|---|---|---|---|---|---|---|
| Sample | On 20 | On 40 | On 100 | On 200 | On 325 | T325 |
| 1* | N | 0.6 | 56.9 | 17.6 | 7.7 | 17.35 |
| 2* | N | 1.7 | 45.4 | 21.3 | 10.6 | 21.3 |

* Average of 4 samples.

| Sample | Compressibility (kp) | | | Density g/cc (lbs/ft3) |
|---|---|---|---|---|
| | 1 Ton | 2 Ton | 3 Ton | Loose/Tapped |
| 1* | - | - | - | 0,79/1,09 (49.2/68.4) |
| 2* | 6.4 | 14.7 | 19.6 | 0,75/0,99 (47.0/61.7) |

*Average of 4 samples.

The product identified as Sample 1 was granulated using various binders in a Glatt™ Powder Coater Granulator model GPCG-5, except Example 9 which was not roller compacted prior to granulation. The formulations, granulation conditions, drying conditions and results are shown in the following Table. The amount of TCP per batch was kept constant at 8 kilograms. Except in Example 4, the binders were used at a 5% w/w level based on the amount of TCP. All binders were prepared as 10% aqueous solutions. Inlet temperature of the granulator was set at 60°C, and drying was conducted at 75°C. The following binders were used in the Examples:

| Example 2 | KLUCEL™ EF hydroxypropyl cellulose |
|---|---|
| 3 | METHOCEL®E5 Premium hydroxypropyl methylcellulose |
| 4 | NATROSOL™ 250L hydroxyethyl cellulose |
| 5 | METHOCEL® A15 hydroxypropyl methylcellulose |
| 6 | PLASDONE® K29/32 polyvinylpyrrolidone (PVP K-29/32) |
| 7 | METHOCEL® E15 hydroxypropyl methylcellulose |
| 8 | PLASDONE® K90 polyvinylpyrrolidone (PVP K-90) |
| 9 | KLUCEL™ EF hydroxypropyl cellulose |

Tableting

750 milligrams of each product was blended for 2 minutes with 0.5% magnesium stearate lubricant and compressed separately in a 1,27 cm (1/2 inch) flat-faced punch and die system using a hydraulic press (Fred Carver, Inc. Model C-12). Tablet hardness was determined using a Schleuniger Table Hardness Tester, Model 2E/106. Tablet hardness (average of 3) is given in the following Table in terms of applied force needed to fracture the tablet prepared using the stated tableting pressure.

The following results were obtained:

6

## TABLE I

| Example | 2 | 3 | 4 |
|---|---|---|---|
| **FORMULATION** | | | |
| TCP Wt., g | 8000 | 8000 | 8000 |
| Binder Type | KLUCEL EF | METHOCEL E5 | NATROSOL 250L |
| Binder Wt., g | 400 | 400 | 320 |
| Water, g | 3600 | 3600 | 3600 |
| **BINDER ATOMIZATION:** | | | |
| Spray Rate, ml/min | 300 | 300 | 300 |
| Atom. Pressure, bar | 3 | 3 | 3-4 |
| Nozzle diameter, mm | 1.2 | 1.2 | 2.2 |
| **BATCH CYCLE:** | | | |
| Spraying Stage, min | 16 | 13 | 18 |
| Drying Stage, min | 34 | 27 | 36 |
| **PRODUCT DENSITY:** | | | |
| Loose Bulk, g/cc | .79 | .74 | .73 |
| Tapped* g/cc | .84 | .81 | .80 |
| **PRODUCT MOISTURE, %:** | 2.7 | 2.6 | 2.0 |
| **PRODUCT SIEVING:** | | | |
| Wt. % on 20 mesh | 0 | 0 | 0 |
| 40 mesh | 4 | 22 | 55 |
| 60 mesh | 51 | 59 | 30 |
| 80 mesh | 20 | 11 | 2 |
| 100 mesh | 8 | 3 | 1 |
| 200 mesh | 11 | 4 | 3 |
| 325 mesh | 4 | 1 | 3 |
| -325 mesh | 3 | 0 | 5 |

*Tapped density obtained by tapping 50 times.

7

### TABLE I (CONTINUED)

| Example | 2 | 3 | 4 |
|---|---|---|---|
| **TABLET HARDNESS, kp:** | | | |
| @ 1 MT | 8.3 | 9.3 | 10.1 |
| @ 2 MT | 16.2 | 17.2 | 17.3 |
| **TABLET THICKNESS, mm:** | | | |
| @ 1 MT | 3.62 | 3.64 | 3.59 |
| @ 2 MT | 3.31 | 3.64 | 3.32 |

### TABLE II

| Example | 5 | 6 | 7 |
|---|---|---|---|
| **FORMULATION** | | | |
| TCP Wt., g | 8000 | 8000 | 8000 |
| Binder Type | METHOCEL A15 | PVP K-29/32 | METHOCEL E15 |
| Binder Wt., g | 400 | 400 | 400 |
| Water, g | 3600 | 3600 | 3600 |
| **BINDER ATOMIZATION:** | | | |
| Spray Rate, ml/min | 300 | 200-300 | 300 |
| Atom. Pressure, bar | 3 | 2-3 | 3 |
| Nozzle diameter, mm | 2.2 | 2.2 | 2.2 |
| **BATCH CYCLE:** | | | |
| Spraying Stage, min | 22 | 15 | 33 |
| Drying Stage, min | 14 | 36 | 18 |
| **PRODUCT DENSITY:** | | | |
| Loose Bulk, g/cc | .71 | .85 | .63 |
| Tapped g/cc | .83 | .92 | .71 |
| **PRODUCT MOISTURE, %:** | 2.0 | 2.7 | 1.8 |

## TABLE II (CONTINUED)

| Example | 5 | 6 | 7 |
|---|---|---|---|
| PRODUCT SIEVING: | | | |
| Wt. % on 20 mesh | 0 | 1 | 8 |
| 40 mesh | 8 | 19 | 39 |
| 60 mesh | 49 | 56 | 8 |
| 80 mesh | 13 | 12 | 3 |
| 100 mesh | 4 | 4 | 3 |
| 200 mesh | 9 | 5 | 13 |
| 325 mesh | 9 | 2 | 13 |
| -325 mesh | 9 | 3 | 13 |
| TABLET HARDNESS, kp: | | | |
| @ 1 MT | 7.5 | 6.8 | 8.6 |
| @ 2 MT | 15.8 | 13.3 | 14.4 |
| TABLET THICKNESS, mm: | | | |
| @ 1 MT | 3.72 | 3.54 | 3.46 |
| @ 2 MT | 3.42 | 3.30 | 3.32 |

## TABLE III

| Example | 8 | 9 |
|---|---|---|
| FORMULATION | | (Control) |
| TCP Wt., g | 8000 | 8000 (TCP Powder) |
| Binder Type | PVP K-90 | KLUCEL EF |
| Binder Wt., g | 400 | 400 |
| Water, g | 3600 | 3600 |
| BINDER ATOMIZATION: | | |
| Spray Rate, ml/min | 300 | 300-450 |
| Atom. Pressure, bar | 3 | 3 |
| Nozzle diameter, mm | 1.2 | 1.2 |
| BATCH CYCLE: | | |
| Spraying Stage, min | 18 | 16 |
| Drying Stage, min | 23 | 51 |
| PRODUCT DENSITY: | | |
| Loose Bulk, g/cc | .72 | .53 |
| Tapped    g/cc | .79 | .60 |
| PRODUCT MOISTURE, %: | 1.8 | 11.0 |
| PRODUCT SIEVING: | | |
| Wt. % on 20 mesh | 17 | 0 |
| 40 mesh | 47 | 2 |
| 60 mesh | 22 | 8 |
| 80 mesh | 5 | 14 |
| 100 mesh | 2 | 25 |
| 200 mesh | 4 | 18 |
| 325 mesh | 2 | 17 |
| -325 mesh | 2 | 16 |

## TABLE III (CONTINUED)

| Example | 8 | 9 |
|---|---|---|
| **TABLET HARDNESS, kp:** | | |
| @ 1 MT | 8.9 | (Product could not |
| @ 2 MT | >18.4 | be tableted) |
| **TABLET THICKNESS, mm:** | | |
| @ 1 MT | 3.69 | |
| @ 2 MT | 3.40 | |

Roller compacted TCP fluidized more easily than powdered TCP. Regular TCP is fluffy, dusty and more difficult to transport. The coarse particles of the roller compacted TCP appeared to assist in particle size build-up. The granulated roller compacted TCP was more easily dried than granulated TCP (1.8-2.7 vis-a-vis 11% moisture in Example 9). A larger amount of roller compacted TCP can be processed per batch due to its higher bulk density. TCP powder was difficult to granulate (mainly -325 mesh and bulk density of 0.3 g/cc). TCP powder was difficult to wet and dry. The wet granulated tricalcium phosphate compositions of the invention (Examples 2, 3, 6) compacted well in a high speed tableting machine (Manesty EB3) to give tablets that had shiny surfaces, and were free of picking. Tablets prepared with roller compacted tricalcium phosphate can show evidence of picking particularly when using an engraved tablet tooling roller, compacted tricalcium phosphate generally leaves a moderate to heavy coating on punch tips. The particles of Example 8 were too large to provide good flow into the tableting dies of the high speed multiple punch tableting machine (Manesty B3B). In Example 9, the drying step took 51 minutes or approximately twice as long as the products prepared in accordance with the invention. After the extensive drying, the product still contained 11% moisture viv-a-vis less than 3% for products of the invention which was unacceptable. The products of the invention are characterized by shorter drying times to a more complete dryness. It appeared that a lot of binder was absorbed by the TCP powder before granulation occurred. Granulated TCP densified by roller compaction as in the invention forms tablets of acceptable strength easily. Tablets could not be made from granulated powdered TCP due to its high level of fines. Also, flow problems were also noted in the product of Example 9 due to the high level of fines.

EXAMPLES 10-18

Roller compacted tricalcium phosphate (Sample 2) was wet granulated in accordance with the procedure of Examples 6 and 8 using polyvinylpyrrolidone as binder. In some examples a tablet disintegrant (Polyplasdone™ crossprovidone XL - a crosslinked polyvinylpyrrolidone) was also added to the TCP prior to wet granulation.

The following results were obtained:

## TABLE IV

| Example | 10 | 11 | 12 |
|---|---|---|---|
| **FORMULATION:** | | | |
| Dry Mix: TCP kg | 12.0 | 12.0 | 9.2 |
| Tab. Dis., kg | 0 | 0 | 2.4 |
| Binder: Type of PVP | K-90 | K-90 | K-90 |
| PVP, kg | .3 | .6 | .5 |
| Water, kg | 2.7 | 5.4 | 4.1 |
| Ratio: PVP/TCP, % | 2.5 | 5.0 | 4.9 |
| Tab. Dis./Product, % | 0 | 0 | 20.0 |
| **RUN CONDITIONS:** | | | |
| Binder Spray Rate, g/min | 300 | 125-250 | 125-250 |
| Atomization Air Pressure, bar | 3 | 3 | 3 |
| Nozzle Orifice, mm | 2.2 | 2.2 | 2.2 |
| Inlet Air Temp. Set-point, $^{\circ}$C | | | |
| During Granulation | 60 | 60 | 60 |
| During Drying | 75 | 75 | 75 |
| **PRODUCT MOISTURE: %** | 2.3 | 2.1 | 2.8 |
| **PRODUCT BULK DENSITY, g/cc** | | | |
| Loose | .76 | .69 | .44 |
| Tapped | .85 | .75 | .51 |
| **PRODUCT SIEVING: Wt. % on** | | | |
| 20 mesh | 4 | 8 | 4 |
| 40 mesh | 17 | 44 | 27 |
| 60 mesh | 39 | 32 | 36 |
| 80 mesh | 15 | 7 | 13 |
| 100 mesh | 7 | 1 | 5 |
| 200 mesh | 7 | 2 | 8 |
| 325 mesh | 4 | 3 | 4 |
| -325 mesh | 7 | 3 | 3 |

## TABLE V

| Example | 13 | 14 | 15 |
|---|---|---|---|
| **FORMULATION:** | | | |
| Dry Mix: TCP kg | 4.8 | 12.0 | 12.0 |
|   Tab. Dis., kg | 5.0 | 0 | 0 |
| Binder: Type of PVP | K-90 | K-29/32 | K-29/32 |
|   PVP, kg | .4 | .6 | .9 |
|   Water, kg | 3.2 | 5.4 | 8.1 |
| Ratio: PVP/TCP, % | 7.5 | 5.0 | 7.5 |
|   Tab. Dis./Product, % | 49.4 | 0 | 0 |
| **RUN CONDITIONS:** | | | |
| Binder Spray Rate, g/min | 125-250 | 300 | 200-300 |
| Atomization Air Pressure, bar | 3 | 3 | 3 |
| Nozzle Orifice, mm | 2.2 | 2.2 | 2.2 |
| Inlet Air Temp. Set-point, $^{\circ}$C | | | |
|   During Granulation | 60 | 60 | 60 |
|   During Drying | 75 | 75 | 75 |
| **PRODUCT MOISTURE: %** | 3.3 | 1.3 | 2.3 |
| **PRODUCT BULK DENSITY, g/cc** | | | |
| Loose | .29 | .92 | .87 |
| Tapped | .34 | 1.02 | .96 |
| **PRODUCT SIEVING: Wt. % on** | | | |
|   20 mesh | 5 | 0 | 2 |
|   40 mesh | 24 | 4 | 12 |
|   60 mesh | 32 | 43 | 38 |
|   80 mesh | 13 | 20 | 16 |
|   100 mesh | 6 | 10 | 8 |
|   200 mesh | 11 | 13 | 16 |
|   325 mesh | 5 | 5 | 4 |
|   -325 mesh | 5 | 6 | 3 |

## TABLE VI

| Example | 16 | 17 | 18 |
|---|---|---|---|
| **FORMULATION:** | | | |
| Dry Mix: TCP kg | 7.4 | 3.6 | 2.7 |
|         Tab. Dis., kg | 2.0 | 4.0 | 3.0 |
| Binder: Type of PVP | K-29/32 | K-29/32 | K-29/32 |
|       PVP, kg | .6 | .4 | .3 |
|       Water, kg | 5.0 | 3.3 | 2.5 |
| Ratio: PVP/TCP, % | 7.5 | 10.0 | 10.0 |
|      Tab. Dis./Product, % | 20.0 | 50.0 | 50.0 |
| **RUN CONDITIONS:** | | | |
| Binder Spray Rate, g/min | 300 | 300 | 200-300 |
| Atomization Air Pressure, bar | 3 | 3 | 3 |
| Nozzle Orifice, mm | 2.2 | 2.2 | 2.2 |
| Inlet Air Temp. Set-point, °C | | | |
|   During Granulation | 60 | 60 | 60 |
|   During Drying | 75 | 75 | 75 |
| **PRODUCT MOISTURE: %** | | 5.2 | 4.4 |
| **PRODUCT BULK DENSITY, g/cc** | | | |
| Loose | .62 | .34 | .33 |
| Tapped | .70 | .39 | .38 |
| **PRODUCT SIEVING: Wt. % on** | | | |
|   20 mesh | 2 | 1 | 0 |
|   40 mesh | 3 | 2 | 2 |
|   60 mesh | 22 | 21 | 20 |
|   80 mesh | 14 | 19 | 18 |
|   100 mesh | 9 | 12 | 14 |
|   200 mesh | 26 | 30 | 33 |
|   325 mesh | 14 | 10 | 10 |
|  -325 mesh | 10 | 6 | 3 |

The wet granulated products from the above examples provided a particle size distribution which can be effectively tableted by direct compression tableting. Using different spray rates, the PVP K-90 level was tested at 2.5% and 5.0%. The K-20/32 level was tested at 5.0% and 7.5%. It appeared that 2.5% K-90 or 5.0% K-29/32 were the respective minimum use levels.

Crosprovidone tablet disintegrant made up 20% of the formulations in Examples 12 and 16. Example 16, which used the K-29/32 as the binder, failed to granulate sufficiently. Example 12 (PVP K-90) appeared to work well.

At 50% tablet disintegrant (crosprovidone) levels (Examples 13, 17 and 18) product handling was very difficult due to the swelling and fluffiness of the disintegrant particles. These batches were difficult to dry and yield losses due to fines resulted. In Examples 16, 17 and 18, excessive fines in the product may be due to the use of PVP K-29/32, which appeared to be a weak binder for crosprovidone. All products containing crosprovidone were low in bulk density which can significantly reduce machine capacity.

EXAMPLES 19-24

Roller compacted tricalcium phosphate from Sample 2 was wet granulated in accordance with the procedure of Examples 6 and 8 using polyvinylpyrrolidone as binder and a 220 liter fluid bed granulator (Glatt GPCG 60). A tablet disintegrant was used as in Examples 12, 13 and 16 - 18. The following results were obtained:

## TABLE VII

| Example | 19 | 20 | 21 |
|---|---|---|---|
| FORMULATION: | | | |
| Dry Mix: TCP kg | 120.0 | 91.5 | 66.0 |
| Tab. Dis., kg | 0 | 24.0 | 30.0 |
| Binder: Type of PVP | K-90 | K-90 | K-90 |
| PVP, kg | 6.0 | 4.5 | 4.0 |
| Water, kg | 54.0 | 40.5 | 35.6 |
| Ratio: PVP/TCP, % | 5.0 | 4.9 | 6.0 |
| Tab. Dis./Product, % | 0 | 20.0 | 30.0 |
| RUN CONDITIONS: | | | |
| Binder Spray Rate, kg/min | 1.3-2.5 | 1.3-2.5 | 1.3-2.5 |
| Atomization Air Pressure, bar | 3 | 3 | 3 |
| Nozzle Orifice, mm | 2.2 | 2.2 | 2.2 |
| # of Nozzles | 3 | 3 | 3 |
| Inlet Air Temp. Set-point, $^{\circ}$C. | | | |
| During Granulation | 60 | 60 | 60 |
| During Drying | 75 | 75 | 75 |
| PRODUCT MOISTURE: % | 2.2 | 2.5 | 2.2 |
| PRODUCT BULK DENSITY, g/cc | | | |
| Loose | .73 | .47 | .39 |
| Tapped | .83 | .56 | .46 |
| PRODUCT SIEVING: | | | |
| Wt. % of 20 mesh | 3 | 4 | 2 |
| 40 mesh | 25 | 20 | 16 |
| 60 mesh | 39 | 33 | 33 |
| 80 mesh | 14 | 15 | 16 |
| 100 mesh | 5 | 7 | 6 |
| 200 mesh | 9 | 12 | 13 |
| 325 mesh | 3 | 5 | 8 |
| -325 mesh | 3 | 5 | 7 |

## TABLE VIII

| Example | 22 | 23 | 24 |
|---|---|---|---|
| FORMULATION: | | | |
| Dry Mix: TCP kg | 120.0 | 74.4 | 64.2 |
| Tab. Dis., kg | 0 | 20.0 | 30.0 |
| Binder: Type of PVP | K-29/32 | K-29/32 | K-29/32 |
| PVP, kg | 7.2 | 5.6 | 5.8 |
| Water, kg | 64.8 | 55.8 | 63.6 |
| Ratio: PVP/TCP, % | 6.0 | 7.5 | 9.0 |
| Tab. Dis./Product, % | 0 | 20.0 | 30.0 |
| RUN CONDITIONS: | | | |
| Binder Spray Rate, kg/min | 1.5-3.0 | 3.0 | 3.0 |
| Atomization Air Pressure, bar | 3 | 3 | 3 |
| Nozzle Orifice, mm | 1.8 | 2.2 | 2.2 |
| # of Nozzles | 6 | 3 | 3 |
| Inlet Air Temp. Set-point, $^{\circ}$C. | | | |
| During Granulation | 60 | 60 | 60 |
| During Drying | 75 | 75 | 75 |
| PRODUCT MOISTURE: % | 2.0 | 2.4 | 2.8 |
| PRODUCT BULK DENSITY, g/cc | | | |
| Loose | .91 | .61 | .50 |
| Tapped | .99 | .71 | .58 |
| PRODUCT SIEVING: | | | |
| Wt. % of   20 mesh | 11 | 5 | 4 |
| 40 mesh | 13 | 5 | 7 |
| 60 mesh | 44 | 31 | 23 |
| 80 mesh | 15 | 15 | 16 |
| 100 mesh | 5 | 8 | 9 |
| 200 mesh | 7 | 21 | 27 |
| 325 mesh | 3 | 8 | 10 |
| -325 mesh | 2 | 6 | 5 |

All six formulations granulated well. The PVP K-90 can be used at a lower usage level and provides a coarser granulation than PVP K-29/32. The product granulated with PVP K-90 seemed to have a narrower particle size distribution, a lower bulk density than the products prepared with PVP K-29/32. High yields were achieved. The product moisture content was controlled within the range of about 2.0-2.8%.

Roller compacted tricalcium phosphate was tableted with the addition of 10% and 20% of the products of Examples 19 - 23. The compositions were tableted in accordance with the procedure of Example 2 using 2.0% Ac-Di-Sol and 0.5% magnesium stearate as lubricant. The die was 1,11 cm (7/16 inch), standard cup with an applied force of 20-23 Kilonewtons. The following results were obtained.

## TABLE IX

| Product of Example | 19 | 19 | 20 | 20 |
|---|---|---|---|---|
| Binder in Tablet | K-90 | K-90 | K-90 | K-90 |
| Tab. Dis. Prod. % | 0 | 0 | 20 | 20 |
| Percent in Tablet | 10 | 20 | 10 | 20 |
| TCP % | 87.5 | 77.5 | 87.5 | 77.5 |
| Applied Force (kN) | 21.8 | 23.0 | 22.1 | 21.2 |
| Crushing Strength (KP) | 12.1 | 12.6 | 13.0 | 13.5 |
| Friability (%) | 0.35 | 0.23 | 0.18 | 0.09 |
| Disintegration (Min.) | 14.8 | 7.8 | 1.3 | 0.9 |

## TABLE IX (CONTINUED)

| Product of Example | 22 | 22 | 23 | 23 |
|---|---|---|---|---|
| Binder | K-29/32 | K-29/32 | K-29/32 | K-29/32 |
| Tab. Dis. % | 0 | 0 | 20 | 20 |
| Percent in Tablet | 10 | 20 | 10 | 20 |
| TCP % | 87.5 | 77.5 | 87.5 | 77.5 |
| Applied Force (kN) | 22.8 | 20.8 | 21.9 | 21.4 |
| Crushing Strength (KP) | 12.6 | 10.3 | 14.6 | 12.8 |
| Friability (%) | 2.30 | 0.17 | 0.15 | 0.11 |
| Disintegration (Min.) | 1.7 | 1.0 | 1.1 | 0.6 |

## TABLE IX (CONTINUED)

| Product of Example | (Control) | (Control) | (Control) |
|---|---|---|---|
| % AVICEL | 0 | 10% AVICEL | 20% AVICEL |
| TCP % | 97.5 | 87.5 | 77.5 |
| Applied Force (kN) | 19.9 | 21.8 | 22.0 |
| Crushing Strength (KP) | 11.0 | 11.7 | 13.9 |
| Friability (%) | 0.6 | – | – |

All tablets prepared using the compositions of the invention (Examples 19, 20, 22 and 23) were shiny and the punch faces were clean. Control tablets made with roller compacted TCP or with microcrystalline cellulose (AVICEL)® were matted in appearance and a slight film was observed on the punch faces.

Replacement of AVICEL® in a roller compacted TCP tablet formulation by the products of the invention also resulted in a smaller tablet based on the equivalent amount of calcium. The data also suggests that tablets incorporating a minor amount of material wet granulated with crosprovidone may not need additional disintegrants.

**Claims**

1. A process for preparing a wet granulatable compressible tricalcium phosphate which comprises compacting tricalcium phosphate and classifying the compacted tricalcium phosphate to such a particle size that the classified particles have a size within the range of less than about 10% larger than about 420 μm, from 40% to 70% larger than 149 μm and smaller than 420 μm and less than about 25% smaller than about 25 μm.

17

**2.** The process as recited in Claim 1 wherein the tricalcium phosphate is compacted using a roller compactor.

**3.** The process as recited in Claim 1 wherein the classified particles have a size within the range of less than about 5% larger than about 420 $\mu$m, from 45% to 65% larger than 149 $\mu$m and smaller than 420 $\mu$m and less than about 20% smaller than about 25 $\mu$m.

**4.** The process as recited in Claim 1 which further includes the step of wet granulating the classified tricalcium phosphate with a binder.

**5.** The process of Claim 4 wherein at least about 90% of the granules of granulated tricalcium phosphate are greater than about 44 $\mu$m, at least about 80% are greater than about 74 $\mu$m and less than about 10% are greater than about 420 $\mu$m.

**6.** The process according to Claim 5 wherein at least about 95% of the particle size of the granules of tricalcium phosphate range from 44 to 420 $\mu$m.

**7.** The process as recited in Claim 4 wherein said binder is selected fron the group consisting of starches, gums, sythetic polymers, cellulose derivatives and gelatines.

**8.** The process as recited in Claim 4 wherein said binder is polyvinylpyrrolidone or starch.

**9.** The product obtainable by the process of anyone of Claims 1 to 8.

**10.** A process for preparing tablets or dry capsules by direct compression which comprises directly compressing the product of Claim 9.

**11.** A wet granulatable tricalcium phosphate composition having a particle size range of less than about 10% larger than about 420 $\mu$m, from 40% to 70% larger than 149 $\mu$m and smaller than 420 $\mu$m and less than about 25% smaller than about 25 $\mu$m.

**12.** The composition as recited in Claim 11 wherein the particle size ranges from less than about 5% larger than about 420 $\mu$m, from 45% to 65% larger than 149 $\mu$m and smaller than 420 $\mu$m and less than about 20% smaller than about 25 $\mu$m.

**13.** A composition comprising the composition of Claim 11 which has been wet granulated with a binder.

**14.** The composition of Claim 13 wherein at least about 90% of the granules of granulated tricalcium phosphate are greater than about 44 $\mu$m, at least about 80% are greater than about 74 $\mu$m and less than about 10% are greater than about 420 $\mu$m.

**15.** The composition according to Claim 14 wherein at least about 95% of the particle size of the granules of tricalcium phosphate range from 44 to 420 $\mu$m.

**16.** The composition as recited in Claim 13 wherein said binder is selected from the group consisting of starches, gums, synthetic polymers, cellulose derivatives and gelatins.

**17.** The composition as recited in Claim 13 wherein said binder is polyvinylpyrrolidone or starch.

**18.** Tablets or dry capsules prepared by directly compressing the composition of any of the Claims 13 to 17 into a tablet or dry capsule form.

**Patentansprüche**

**1.** Verfahren zur Herstellung eines feuchtgranulierbaren, komprimierbaren Tricalciumphosphats, bei dem Tricalciumphosphat kompaktiert und das kompaktierte Tricalciumphosphat auf eine solche Partikelgröße klassiert wird, daß die klassierten Partikel eine solche Größe besitzen, daß weniger als etwa 10% größer als etwa 420 $\mu$m, 40% bis 70% größer als 149 $\mu$m und kleiner als 420 $\mu$m und weniger als

etwa 25% kleiner als etwa 25 $\mu$m sind.

2. Verfahren nach Anspruch 1, bei dem das Tricalciumphosphat unter Einsatz eines Walzenverdichters kompaktiert wird.

3. Verfahren nach Anspruch 1, bei dem die klassierten Partikel eine solche Größe besitzen, daß weniger als etwa 5% größer als etwa 420 $\mu$m, 45% bis 65% größer als 149 $\mu$m und kleiner als 420 $\mu$m und weniger als etwa 20% kleiner als etwa 25 $\mu$m sind.

4. Verfahren nach Anspruch 1, bei dem das klassierte Tricalciumphosphat außerdem mit einem Bindemittel feuchtgranuliert wird.

5. Verfahren nach Anspruch 4, bei dem mindestens etwa 90% der Körnchen des granulierten Tricalciumphosphats größer als etwa 44 $\mu$m, mindestens etwa 80% größer als etwa 74 $\mu$m und weniger als etwa 10% größer als etwa 420 $\mu$m sind.

6. Verfahren nach Anspruch 5, bei dem mindestens etwa 95% der Partikelgröße der Körnchen des Tricalciumphosphats von 44 bis 420 $\mu$m reicht.

7. Verfahren nach Anspruch 4, bei dem das Bindemittel ausgewählt ist aus der Gruppe bestehend aus Stärken, Gummis, synthetischen Polymeren, Cellulosederivaten und Gelatinen.

8. Verfahren nach Anspruch 4, bei dem das Bindemittel Polyvinylpyrrolidon oder Stärke ist.

9. Das nach dem Verfahren nach einem der Ansprüche 1 bis 8 erhältliche Produkt.

10. Verfahren zur Herstellung von Tabletten oder Trockenkapseln durch direkte Kompression, worin das Produkt des Anspruchs 9 direkt komprimiert wird.

11. Eine feuchtgranulierbare Tricalciumphosphat-Zusammensetzung mit einem Partikelgrößenbereich, bei dem weniger als etwa 10% größer als 420 $\mu$m, 40% bis 70% größer als 149 $\mu$m und kleiner als 420 $\mu$m und weniger als etwa 25% kleiner als etwa 25 $\mu$m ist.

12. Zusammensetzung nach Anspruch 11, bei der die Partikelgröße für weniger als 5% größer als etwa 420 $\mu$m, für 45% bis 65% größer als 149 $\mu$m und kleiner als 420 $\mu$m und für weniger als etwa 20% kleiner als etwa 25 $\mu$m ist.

13. Zusammensetzung, die die Zusammensetzung nach Anspruch 11 enthält, welche mit einem Bindemittel feuchtgranuliert worden ist.

14. Zusammensetzung nach Anspruch 13, bei der mindestens etwa 90% der Körnchen des granulierten Tricalciumphosphats größer als etwa 44 $\mu$m sind, mindestens etwa 80% größer als etwa 74 $\mu$m sind und weniger als etwa 10% größer als etwa 420 $\mu$m sind.

15. Zusammensetzung nach Anspruch 14, bei der mindestens etwa 95% der Partikelgröße der Körnchen des Tricalciumphosphats von 44 bis 420 $\mu$m reicht.

16. Zusammensetzung nach Anspruch 13, worin das Bindemittel ausgewählt ist aus der Gruppe bestehend aus Stärken, Gummis, synthetischen Polymeren, Cellulosederivaten und Gelatinen.

17. Zusammensetzung nach Anspruch 13, bei der das Bindemittel Polyvinylpyrrolidon oder Stärke ist.

18. Tabletten oder Trockenkapseln, hergestellt durch direktes Komprimieren der Zusammensetzung nach einem der Ansprüche 13 bis 17 in eine Tabletten- oder Trockenkapselform.

**Revendications**

1. Un procédé de préparation d'un phosphate tricalcique granulable par voie humide pouvant être

19

comprimé, qui comprend les étapes de: comprimer du phosphate tricalcique et classer le phosphate tricalcique comprimé jusqu'à ce que les particules classées aient la répartition granulométrique suivante: moins d'environ 10% pour une granulométrie supérieure à environ 420 $\mu$m; de 40 à 70% pour une granulométrie supérieure à 149 $\mu$m et inférieure à 420 $\mu$m; et moins d'environ 25% pour une granulométrie inférieure à environ 25 $\mu$m.

2. Le procédé selon la revendication 1, dans lequel le phosphate tricalcique est comprimé à l'aide d'une unité de compression à rouleaux.

3. Le procédé selon la revendication 1, dans lequel les particules classées ont la répartition granulométrique suivante: moins d'environ 5% pour une granulométrie supérieure à environ 420 $\mu$m; de 45 à 65% pour une granulométrie supérieure à 149 $\mu$m et inférieure à 420 $\mu$m; et moins d'environ 20% pour une granulométrie inférieure à environ 25 $\mu$m.

4. Le procédé selon la revendication 1, qui comprend en outre l'étape consistant à granuler par voie humide, avec un liant, le phosphate tricalcique classé.

5. Le procédé selon la revendication 4, dans lequel au moins environ 90% des granulés de phosphate tricalcique granulé ont une granulométrie supérieure à environ 44 $\mu$m, au moins environ 80% une granulométrie supérieure à environ 74 $\mu$m et moins d'environ 10% d'une granulométrie supérieure à environ 420 $\mu$m.

6. Le procédé selon la revendication 5, dans lequel au moins environ 95% des granulés de phosphate tricalcique ont une granulométrie comprise entre 44 et 420 $\mu$m.

7. Le procédé selon la revendication 4, dans lequel ledit liant est choisi parmi l'ensemble comprenant les amidons, les gommes, les polymères synthétiques, les dérivés de la cellulose et la gélatine.

8. Le procédé selon la revendication 4, dans lequel ledit liant est la polyvinylpyrrolidone ou l'amidon.

9. Le produit pouvant être obtenu par le procédé selon l'une quelconque des revendications 1 à 8.

10. Un procédé de préparation de comprimés ou de gélules sèches par compression directe, qui consiste à comprimer directement le produit selon la revendication 9.

11. Une composition de phosphate tricalcique granulable par voie humide ayant une répartition granulométrique telle qu'environ 10% des particules ont une granulométrie supérieure à environ 420 $\mu$m, 40 à 70% ont une granulométrie supérieure à 149 $\mu$m et inférieure à 420 $\mu$m, et moins d'environ 5% ont une granulométrie supérieure à environ 25 $\mu$m.

12. La composition selon la revendication 11, dans laquelle la répartition granulométrique est telle qu'environ 5% des particules ont une granulométrie supérieure à environ 420 $\mu$m, 45 à 65% ont une granulométrie supérieure à 149 $\mu$m et inférieure à 420 $\mu$m, et moins d'environ 20% ont une granulométrie supérieure à environ 25 $\mu$m.

13. Une composition comprenant la composition selon la revendication 11, qui a été granulée par voie humide à l'aide d'un liant.

14. La composition selon la revendication 13, dans laquelle au moins environ 90% des granulés de phosphate tricalcique granulé ont une granulométrie supérieure à environ 44 $\mu$m, au moins environ 80% une granulométrie supérieure à environ 74 $\mu$m et moins d'environ 10% d'une granulométrie supérieure à environ 420 $\mu$m.

15. La composition selon la revendication 14, dans laquelle au moins environ 95% des granulés de phosphate tricalcique granulé ont une granulométrie comprise entre 44 et 420 $\mu$m.

16. La composition selon la revendication 13, dans laquelle ledit liant est choisi parmi l'ensemble comprenant les amidons, les gommes, les polymères synthétiques, les dérivés de la cellulose et la gélatine.

**17.** La composition selon la revendication 13, dans laquelle ledit liant est la polyvinylpyrrolidone ou l'amidon.

**18.** Comprimés ou gélules sèches préparés par compression directe de la composition selon l'une quelconque des revendications 13 à 17, en une forme de comprimé ou gélule sèche.